# FASCICULE DE BREVET EUROPEEN

(11) **EP 0 814 759 B1**
(45) Date de publication et mention de la délivrance du brevet: **30.05.2001**
(21) Numéro de dépôt: 96907532.4
(22) Date de dépôt: 14.03.1996
(51) Int. Cl.: A61K 7/42

(54) **COMPOSITIONS ECRAN-SOLAIRE COMPRENANT UN MELANGE DE PARTICULES D'OXYDE DE TITANE ET DE ZINC, LEUR PROCEDE DE PREPARATION ET LEUR UTILISATION**
SONNENSCHUTZMITTEL ENTHALTEND EINE MISCHUNG AUS TITANOXYD - UND ZINCOXYDTEILCHEN, DEREN HERSTELLUNGSVERFAHREN UND DEREN VERWENDUNG
SUNSCREEN COMPOSITIONS COMPRISING A MIXTURE OF TITANIUM AND ZINC OXIDE PARTICLES, METHOD FOR PREPARING THE SAME AND USE THEREOF

(30) Priorité: 15.03.1995 FR 9502997
(43) Date de publication de la demande: 07.01.1998
(73) Titulaire: Pierre Fabre Dermo-Cosmetique, 92100 Boulogne (FR)
(72) Inventeur: MSIKA, Philippe, F-31000 Toulouse (FR); CARRIERE, Francis, F-31320 Castanet-Tolosan (FR); FABRE, Jean-Pierre, F-81100 Castres (FR); BOYER, France, F-31320 Pechabou (FR)
(74) Mandataire: Ahner, Francis
(86) Numéro de dépôt international: FR9600393
(87) Numéro de publication internationale: WO9628136

(56) Documents cités:
- EP-A- 0 433 086
- EP-A- 0 456 460
- EP-A- 0 535 972
- WO-A-90/11067
- GB-A- 2 184 356

## Description

L'invention a pour objet des compositions écran-solaire à base d'oxyde de titane et/ou d'oxyde de zinc. Elle vise également leur procédé d'obtention et leur utilisation cosmétique et thérapeutique pour la protection de la peau contre tous les effets des rayonnements ultraviolets A et B.

Il est connu d'utiliser de l'oxyde de titane (TiO₂) et/ou de l'oxyde de zinc (ZnO) comme agents réfléchissants des rayons ultraviolets.

A titre d'exemples, les brevets EP 91 304 100, GB 87/17662 ou WO 89/01438 décrivent des compositions écran-solaire comportant de l'oxyde de titane comme agent protecteur contre les rayonnements.

De la même façon, les brevets EP 535 972 et WO 92/13517 décrivent des compositions écran-solaire comportant de l'oxyde de zinc comme agent protecteur contre les rayonnements.

Les brevets FR 2 591 480 et EP 833 086 décrivent, quant à eux, des compositions écran-solaire comportant une association des deux oxydes. Il apparait que cette association a un effet synergique sur la valeur de l'indice de protection solaire. Cette association se montre, par conséquent, particulièrement avantageuse pour l'obtention de compositions écran-solaire.

Cependant, les compositions ainsi obtenues pourraient encore être améliorées. En effet, il serait bénéfique d'augmenter leur stabilité et surtout l'homogénéité de la dispersion des particules. On pourrait également optimiser encore leur pouvoir protecteur.

L'invention a pour but de fournir une composition écran-solaire à base d'oxyde de titane et/ou d'oxyde de zinc, de meilleure qualité que celles qui existent déjà, en ce qui concerne :
- leur stabilité dans le temps,
- l'homogénéité de leur dispersion dans la formule et sur la peau,
- la non-réagglomération des cristaux dans le temps, et
- leur pouvoir protecteur.

On y parvient, selon l'invention, en mettant en oeuvre des particules d'oxyde de zinc et des particules d'oxyde de titane, empâtées dans des huiles et/ou esters et/ou alcools gras et/ou éthérs d'empâtage sélectionnés dans le but d'obtenir :
- un point de mouillage des particules d'oxyde de titane empâtées et un point de mouillage des particules d'oxyde de zinc empâtées, environ inférieurs à 5,
- un point de fluidité des particules d'oxyde de titane empâtées et un point de fluidité des particules d'oxyde de zinc empâtées, environ inférieurs à 30.

On appelle point de mouillage, respectivement point de fluidité des particules empâtées, la quantité en poids de vaseline à ajouter à un mélange de 9 g de particules et 7 g d'huile d'empâtage, pour obtenir le mouillage complet des poudres, respectivement une pâte fluide.

On entend par empâtage des particules de TiO₂, l'opération consistant à prédisperser, par broyage, lesdites particules dans un choix d'huiles et/ou d'esters et/ou d'alcools gras et/ou d'éthers aux qualités hyperdispersantes, de telle sorte qu'après broyage au tricylindre ou au broyeur à bille des particules et des huiles et/ou des esters et/ou des alcools gras et/ou des éthers, on obtienne une pâte blanche, stable, pouvant être incluse directement dans le processus industriel. Avec l'empâtage, on évite toute réagglomération des particules pouvant être responsable de la baisse de la photoprotection sur la peau du produit fini.

Les particules ainsi prédispersées dans ce mélange d'huiles et/ou d'esters et/ou d'alcools gras et/ou d'éthers, sans émulsionnant, passent de l'état de motte et d'agglomérat (de plus de 100 microns) à une forme monodispersée et monocristalline idéale (15 à 30 nm).

En ce qui concerne le TiO₂, cette pâte prête à l'emploi pour le processus industriel est différente des poudres d'oxyde de titane et des suspensions commerciales telles que le TioVeil (Tioxyde) : la pâte blanche est obtenue sans adjonction de composés annexes tensio-actifs et de composés organiques.

L'empâtage des particules de ZnO est de même nature que celle de TiO₂.

On pourra aussi mettre en oeuvre des particules d'oxyde de zinc, telles que par exemple celles de type Spectraveil, commercialisées par Tioxyde, sans jamais l'associer au TIOVEIL (TIOXYDE), suspension d'oxyde de titane aciculaire.

L'association des deux oxydes empâtés dans des huiles et/ou des esters et/ou des alcools gras et/ou des éthers conformément à l'invention conduit à un effet synergique sur l'indice de protection alors que la même association sans empâtage ne donne pas lieu à cette synergie.

Avantageusement, les particules d'oxyde de titane présentent une taille inférieure ou égale à 20 ± 5 nm et les particules d'oxyde de zinc, une taille supérieure ou égale à 60 ± 5 nm.

On pourra utiliser, notamment, des cristaux de TiO₂ de type rutile, de morphologie prismatique et non aciculaires, par exemple :
- T 805, commercialisés par Degussa (traité alkyl silane), et
- MT 100T, commercialisé par Tayca (enrobé acide stéarique et hydroxyde d'aluminium).

On pourra utiliser, comme particules de ZnO, notamment des particules d'oxyde de zinc ultrafines en poudre, du type Z-côte et Z-côte HP1 (sunsmart) ou zinc neutral (H & H) ou éventuellement une suspension huileuse d'oxyde de zinc Spectraveil (TIOXYDE).

De préférence, les particules d'oxyde de titane présentent une taille moyenne égale à 20 nm et les particules d'oxyde de zinc, une taille moyenne égale à 60 nm.

Selon un mode de réalisation avantageux de la présente invention, les huiles et/ou esters et/ou alcools gras et/ou éther d'empâtage sont sélectionnés dans le groupe comprenant le décyl oléate, un C₁₂₋₁₅ alkyl benzoate, l'octyl dodécanol, l'octyl dodécyl néopentanoate, le glycéryl oléate et le propylène glycol, le propylène glycol dioctanoate, les triglycérides caprique/caprylique, le cétéaryl octanoate, l'octyl palmitate, l'isoarachidyl néopentanoate, le dioctyl maléate, le dicapryléther, ainsi que leurs mélanges.

On choisira, de préférence d'empâter les particules d'oxyde de titane dans un mélange de décyl oléate et de C₁₂₋₁₅ alkyl benzoate et les particules d'oxyde de zinc dans des triglycérides caprique/caprylique.

Par exemple, on mélangera 50 % en poids de particules de TiO₂, 30 % en poids de Finsolv TN et 20 % en poids de Cetiol V (HENKEL).

Avantageusement, l'oxyde de titane est présent dans l'empâtage à une concentration relative en poids comprise entre 20 et 60 % et dans la formulation finale au maximum de 25 %.

Plus avantageusement encore, l'oxyde de zinc est présent dans l'empâtage à une concentration relative en poids comprise entre 20 et 60%. La quantité finale dans la formulation sera inférieure à 30 %.

Selon un autre mode préférentiel de réalisation de la composition selon l'invention, celle-ci comporte en outre des particules d'oxyde de fer pigmentaire de taille égale à 1 à 10 µm ou sous forme nanomisée type Nassy Cat de LACHI Inc. (USA) < 50 nm, leur proportion relative en poids étant comprise entre 0,1 et 3 %.

L'adjonction d'oxyde de fer a pour effet de mettre à la teinte la formule dans les indices les plus élevés (IP 30; 40; 50), en apportant de la couleur jaune et rouge afin de rendre transparent le produit étalé sur la peau et de ne pas avoir de blancheur incompatible avec l'utilisation ainsi que de protéger fortement dans les I.R. notamment.

Le procédé de fabrication d'une composition écran-solaire selon l'invention comporte une étape préliminaire consistant à :
- empâter des particules d'oxyde de titane dans au moins une huile et/ou un ester et/ou un alcool gras et/ou un éther d'empâtage, afin d'obtenir des particules d'oxyde de titane empâtées, et
- empâter des particules d'oxyde de zinc dans au moins une huile et/ou un ester et/ou un alcool gras et/ou un éther d'empâtage afin d'obtenir des particules d'oxyde de zinc empâtées, éventuellement
- mélanger les particules d'oxyde de titane empâtées et les particules d'oxyde de zinc empâtées, afin d'obtenir une pâte destinée à être incorporée dans ladite composition.

Pour obtenir les particules d'oxyde de titane empâtées, on pourra, par exemple, utiliser des particules sèches d'oxyde de titane, les incorporer dans une huile et/ou un ester et/ou un alcool gras et/ou un éther, mélanger l'ensemble grossièrement et passer le tout une à trois fois au broyeur cylindrique. Les particules d'oxyde de titane empâtées se présentent, dans ce cas, sous la forme d'une pâte blanche, homogène et brillante.

Pour obtenir les particules d'oxyde de zinc empâtées, on pourra procéder de manière analogue.

Selon un mode de mise en oeuvre préférentiel du procédé selon l'invention, l'huile et/ou l'ester et/ou l'alcool gras et/ou l'éther d'empâtage des particules d'oxyde de titane et l'huile et/ou l'ester et/ou l'alcool gras et/ou l'éther d'empâtage des particules d'oxyde de zinc sont sélectionnés indépendamment l'un de l'autre, afin d'obtenir :
- un point de mouillage des particules d'oxyde de titane empâtées et un point de mouillage des particules d'oxyde de zinc empâtées environ inférieurs à 5, et
- un point de fluidité des particules d'oxyde de titane empâtées et un point de fluidité des particules d'oxyde de zinc empâtées environ inférieurs à 30.

Avantageusement, la composition se présente sous la forme d'une émulsion eau dans huile, en particulier une émulsion eau dans l'huile à base de silicone.

On pourra ajouter des dérivés gélifiants d'origine minérale du groupe des Montmorillonites du type Bentone (38;34) (Quarternium 14, 18 Hectorite) ou d'aérosil (Degussa par exemple Aérosil R 972), qui judicieusement introduits lors du processus industriel permettent d'optimiser la stabilité du produit. De plus, ils potentialisent fortement la protection solaire dans les indices de protection les plus forts et stabilisent l'indice de protection au cours du temps.

La composition selon l'invention est introduite dans un véhicule. Il agit comme diluant dispersant, transporteur de matériaux photoprotecteurs (oxydes métalliques + gélifiants minéraux) relatés ci-dessus et il doit faciliter leur distribution sur la peau humaine en respectant la dispersion monocristalline.

Ce véhicule est ici, essentiellement, à phase lipophile continue, émulsion ou excipient anhydre.

La composition selon l'invention peut se présenter sous forme de suspension ou de dispersion dans des solvants ou des corps gras, sous forme d'émulsion telle qu'une crème ou un lait, sous forme d'une pommade, d'un gel, d'un composé solide anhydre ou non, ou être conditionnée en aérosol et se présenter sous forme de mousse.

Elle peut contenir les adjuvants usuels dans ce type de composition, tels que des épaississants, des adoucissants, des produits hydratants, des tensio-actifs, des conservateurs, des séquestrants, des antioxydants, des anti-mousses, des huiles, des cires, de la lanoline, des parfums, des propulseurs, des colorants, des vitamines ou tout autre ingrédient habituellement utilisé.

Dans le cas d'une composition conditionnée en aérosol, on utilise des propulseurs classiques tels que les alcanes, les fluoro-alcanes et les chlorofluoroalcanes, le diméthyl éther ou l'oxyde nitreux.

Parmi les principaux adjuvants pouvant être présents dans les compositions de l'invention, on peut citer les solvants tels que l'eau, les monoalcools ou les polyols inférieurs contenant 1 à 6 atomes de carbone ou leurs mélanges, les monoalcools ou polyols, l'éthanol, l'isopropanol, le propylèneglycol, la glycérine et le sorbitol; on peut également citer les corps gras tels que les huiles ou cires minérales, animales, végétales ou synthétiques, les acides gras, les esters d'acides gras tels que les triglycérides d'acide gras ayant de 6 à 12 atomes de carbone, les alcools gras, la vaseline, la paraffine, la lanoline, la lanoline hydrogénée, la lanoline acétylée, l'huile de silicone.

Mais aussi des poudres talc, terres diverses, kaolins, amidon, gommes végétales ou synthétique, nylon, smectites et dérivés, polyacrylate et dérivés, silicates de magnésium ou d'aluminium modifiés ou non, xanthane et dérivés, polymère de carboxyvinyl et dérivés ou encore des conservateurs comme les parabens par exemple, des antioxydants comme le B.H.T. par exemple, des parfums, des masqueurs d'odeur, des extraits actifs divers, des colorants peuvent être mis en oeuvre.

On pourra réaliser une émulsion du type eau-dans-l'huile. Elle comporte une phase aqueuse, une phase grasse et un système émulsionnant.

Dans ce type d'émulsion, la concentration en système émulsionnant est comprise entre 4 et 35% en poids par rapport au poids total de l'émulsion; la phase grasse est présente en des proportions comprises entre 20 et 60% en poids et la phase aqueuse en des proportions comprises entre 20 et 70% en poids par rapport au poids total de l'émulsion. Les émulsionnants sont ceux utilisés habituellement dans ce type d'émulsion.

La phase grasse peut également contenir des huiles de silicone solubles dans les autres huiles, tels que le diméthylpolysiloxane, le méthyl phényl polysiloxane et les copolymères silicone-glycol, des acides gras et des alcools gras.

En vue de favoriser la rétention des huiles, on peut également utiliser des cires telles que la cire de carnauba, la cire de candellila, la cire d'abeille, la cire microcristalline, l'ozokérite, les oléates, les myristates, les linoléates et les stéarates de Ca, Mg et Al.

Les émulsions du type eau-dans-l'huile peuvent se présenter sous forme de sticks. Dans ce cas, la concentration de la phase aqueuse dans l'émulsion est généralement comprise entre 5 et 70% en poids par rapport au poids total de l'émulsion.

Généralement, ces émulsions eau-dans-l'huile sont préparées en introduisant dans une cuve de fabrication la phase grasse et l'émulsifiant. On chauffe à une température de 70-75°C. On ajoute ensuite les ingrédients solubles dans l'huile puis on ajoute, en agitant, l'eau portée préalablement à la même température, eau dans laquelle on a préalablement dissous les ingrédients hydrosolubles; on agite jusqu'à obtention d'une émulsion ayant la finesse désirée, puis on laisse refroidir à température ambiante, éventuellement sous agitation lente.

Les gels gras comprennent une huile ou cire et un épaississant tel que la silice. Les gels oléoalcooliques ou hydroalcooliques comprennent un ou plusieurs alcools ou polyols inférieurs comme l'éthanol, le propylèneglycol ou la glycérine, un épaississant tel que la silice, les dérivés de cellulose, les dérivés d'acide polyacryliques, les gommes de guar, de caroube et de xanthane, en présence d'huile ou d'eau respectivement.

Les sticks et autres formes anhydres solides sont constitués de corps gras comme les cires et huiles naturelles ou sythétiques, les alcools gras, les esters d'acides gras et la lanoline.

Quant aux émulsions eau-dans-silicone, elles peuvent notamment être formulées à base :
- d'émulsionnants siliconés, issus de polymères de haut poids moléculaire,
- de polymère de diméthyl polysiloxane avec des chaînes polyoxyéthylène et/ou polyoxypropylène ayant un poids moléculaire de 10 000 à 50 000.

Le polymère de diméthyl polysiloxane est souvent dispersé dans du silicone volatil. Cette dispersion comprend, par exemple, de 1 à 20% en volume en polymère et de 80 à 99% en silicone volatil.

Parmi ces émulsionnants, on peut citer le Dow 322 5C, le Q2 5200 et leurs dérivés, par exemple.

Parmi une autre famille d'émulsionnants siliconés, les polysiloxanes polyalkyl polyéther peuvent être mis en oeuvre, par exemple le copolyolcétyl diméthicone (Abil EM 90) et ses dérivés (Abil WE 09 et Abil WS 08) (GOLDSCHMIDT), seuls ou associés à un émulsionnant eau/huile non ionique de type ester de polyglycérol.

La composition pourra comprendre, en outre, des photoprotecteurs, des anti-oxydants et antiradicalaires et des adjuvants autres.

On citera, comme anti-oxydants et antiradicalaires par exemple : la vitamine E et ses acétates, la vitamine A et ses dérivés, le β-carotène, le rétinol, la vitamine C et ses dérivés, le glutathion, le sélénium, les oligo-éléments divers, le B.H.T., le B.H.A., les flavonoïdes et polyphénols d'origine végétale par exemple, la mélanine et précurseurs d'origine humaine, végétale, animale ou biotechnologique.

On citera par ailleurs, comme adjuvants :
- toutes les huiles végétales pouvant apporter un bénéfice au niveau de la formulation solaire par absorption des UV (sésame, olive, pépins de raisin, coco...).
- tous les composés filmogènes permettant la bonne rémanence à l'eau et à la sueur du produit au cours du temps (dérivés de P.V.P., Pémulen et dérivés (Goodrich), cire de silicone, silicone rémanent à l'eau type DOW 593; Antaron (WP660; V 220); Gantrez E 542S, dérivés cellulosiques).

Les compositions conformes à l'invention peuvent être utiles en cosmétique pour la protection de la peau contre les effets des radiations solaires UV A et UV B à court ou à long terme.

Elles peuvent également être utiles pour la fabrication de médicaments destinés à être utilisés pour des traitements dermatologiques d'affections telles que la Lucite Estivale Bénigne (L.E.B.).

### I - Empâtage

### 1- Méthode

Ci-après est expliquée la manière de choisir l'huile ou l'ester ou l'alcool gras ou l'éther d'empâtage des particules d'oxyde de titane, en mesurant le point de mouillage et le point de fluidité.

On choisit plusieurs huiles ou esters ou alcool gras ou éthers d'empâtage et on évalue pour chacun d'eux ou leurs mélanges les points en question.

Pour cela, on utilise 9 g de particules d'oxyde de titane et 7 g de l'huile ou de l'ester en question. La quantité de vaseline à ajouter (x grammes) pour la détermination des points est reportée dans le tableau ci-après.

On prendra comme référence :
- pour le point de mouillage, 15 g de vaseline,
- pour le point de fluidité, 60 g de vaseline

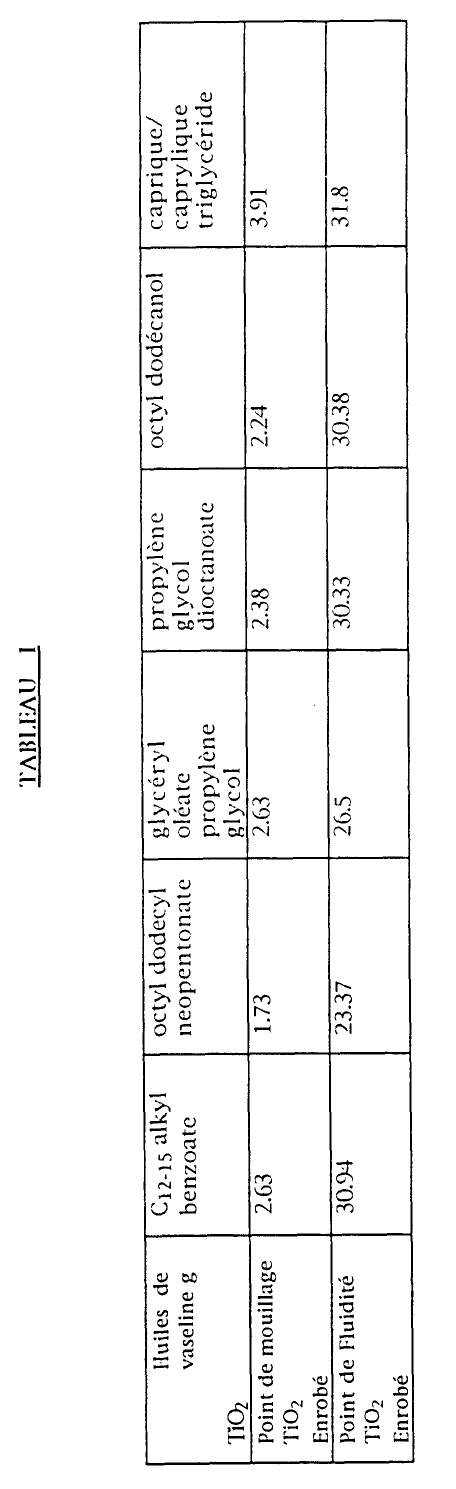

### 2 - Intérêt de l'empâtage

a) Intérêt de la dispersion huileuse mécanique et du choix de l'ester d'empâtage de TiO₂.
On mesure l'indice de protection solaire de compositions à base de particules de TiO₂ en fonction de la nature de l'empâtage. On mesure également le même indice de protection B + A contre les coups de soleil, pour des particules non empâtées.

**TABLEAU II**

| Qualité de TiO₂ | I.P.(B + A) à 5 % | I.P.(B.+ A) à 10 % |
|---|---|---|
| TiO₂ enrobé | 5 | 10 |
| TiO₂ empâté huile de vaseline | 5 | 10 |
| TiO₂ Empâtage 1 | 7 | 14 |
| TiO₂ Empâtage 2 | 10 | 20 |
| Empâtage 1 : octyldodécanol | | |
| Empâtage 2 : décyloléate | | |

Ces mesures montrent l'intérêt de disperser mécaniquement le TiO₂ dans un lipide choisi. La protection est supérieure à celle procurée par des particules non enrobées.
De plus, on observe des différences de dispersion et donc de photoprotection entre les empâtages. L'huile de vaseline est inefficace, l'alcool gras est assez efficace, le décyloléate double la photoprotection.
b) Intérêt de la dispersion des oxydes métalliques
On mesure l'indice de protection de diverses compositions en fonction de l'empâtage des particules de TiO₂ et de ZnO par des empâtages différents.
- 12,5% TiO₂+1,2% ZnO → IP 15 non dispersé
- 12,5 % TiO₂ + 1,2 % ZnO → IP 35 dans Empâtage 1
- 12,5 % TiO₂ + 1,2 % ZnO → IP 70 dans Empâtage 2

c) Effet de la quantité des particules de TiO₂ avec Empâtage 2
On mesure à nouveau l'indice de protection des diverses compositions en fonction de la quantité des particules de TiO₂ empâtées dans le décyloléate.
- 8 % TiO₂ + 1,2 % ZnO → IP 30
- 10 % TiO₂ + 1,2 % ZnO → IP 36
- 12,5 % TiO₂ + 1,2 % ZnO → IP 70

d) Effet de la quantité de ZnO
On mesure une fois de plus l'indice de protection de diverses compositions en fonction de la qualité des particules de ZnO empâtées dans l'Empâtage 2
- 10,5 % TiO₂ + 1,2 % ZnO (Empâtage 2) → IP 36,4
- 10,5 % TiO₂ + 2,4 % ZnO (Empâtage 2) → IP 50

e) Intérêt de Fe₂O 3
L'intérêt de la présence d'oxyde de fer est la mise à la teinte de la peau, des compositions fortement dosées en oxydes métalliques (TiO₂ et ZnO), afin de permettre une transparence immédiate après application sur la peau, pour les produits d'indice extrême.
Pour mesurer la teinte de la peau, on réalise un essai colorimétrique (L, a, b) après application de 2 formules avec et sans oxydes de fer, sur la peau nue.

Formule A : 10,5 % TiO₂ + 2,4 % ZnO

Formule B : 10,5 % TiO₂ + 2,4% ZnO + mélange d'oxydes de fer à 2 %

Il apparaît que la formule B donne à la peau une couleur naturelle proche d'une peau non traitée.
f) Intérêt des minéraux gélifiants
On calcule l'indice de protection solaire d'une composition comportant un gélifiant; les résultats sont les suivants :
Formule avec 0,3 % de Bentone 38 → IP 44
Formule avec 0,8 % de Bentone 38 → IP 55

g) Protection UV A
La protection UV A a été calculée par la méthode de la P.P.D. ou "Permanent Pigmentation Darkening" en évaluant l'inhibition du bronzage immédiat aux UV A courts et longs, d'une peau traitée par la formule photoprotectrice contre le bronzage d'une peau nue avec une lecture du bronzage à 2 heures.
L'irradiation se réalise avec une lampe Robertson Berger, filtrée WG 335 et UG 11.
h) Intérêt de l'association TiO₂ empâté et ZnO
On mesure les indices de protection de formulations comportant, soit des particules de TiO₂ seulement, soit des particules de ZnO seulement, soit un mélange des deux.
Les résultats sont reportés ci-après :
- 10 % TiO₂ en poudre → IP UV A 4
- 2,4% ZnO → IP UV A 2,5
- 10 % TiO₂ empâté/décyloléate +2,4 ZnO → IP UV A 10

On évalue ci-après l'effet de la quantité de chaque poudre sur l'indice de protection :
- 8 % TiO₂/décyloléate + 1,2 % ZnO → IP UV A 6,5
- 10 % TiO₂/décyloléate + 1,2 % ZnO → IP UV A 8,2
- 10 % TiO₂/décyloléate + 2,4 % ZnO → IP UV A 10

### II - Stabilité dans le temps et suivant le procédé

Pour mesurer la stabilité dans le temps des compositions, on procède comme suit :

On conserve à la chaleur à 40°C sur des périodes de 3 mois les formulations à base d'empâtage ou suspension de TiO₂ + ZnO. On calcule l'IP UV B + A à température ambiante, puis après 1 mois et 3 mois à 40°C.

Les résultats sont regroupés dans le tableau III suivant.

**TABLEAU III**

| | TO | T1 mois 40°C | T3 mois 40°C |
|---|---|---|---|
| FR 12 15 | 44±5 | 42±5 | - |
| FR 12 35 - LP | 50 ± 5 | - | 49 ± 5 |

On observe que les compositions selon l'invention sont particulièrement stables.

On évalue ainsi la stabilité sur plusieurs années : 1 mois à 40°C équivaut à 1 an à température ambiante; l'empâtage permet de conserver la dispersion des minéraux dans le temps.

L'invention pourra être mieux comprise à l'aide des exemples non limitatifs qui suivent et qui constituent des modes de réalisation privilégiés de la composition selon l'invention.

### EXEMPLES

On expose dans ce qui suit des exemples de formulations de compositions écran-solaire selon l'invention. Les pourcentages sont des pourcentages en poids.

### 1 - Exemple de formulations

| | |
|---|---|
| DOW CORNING 3225 C | 10,00 g |
| DECAMETHYL CYCLOPENTASILOXANE | 1 à 10 g |
| PATE DE TITANE | 1 à 50 g |
| SPECTRAVEIL MOTG | 0,1 à 20 g |
| VASELINE BLANCHE QUALITE A | 1,000 g |
| BENTONE 38 STERILISEE | 0,1 à 2 g |
| MONOSTEARATE DE GLYCEROL | 0,1 à 2 g |
| MONOMULS 90L12 | 0,1 à 2 g |
| ACETATE D'ALPHA TOCOPHEROL | 0,500 g |
| NaCl SUPER EPURE DESULFATE | 2,000 g |
| KELTROL T.F. | 0,150 g |
| WITCONOL APM | 0,800 g |
| PARAHYDROXY BENZOATES | 0,600 g |
| EUXYL K 400 | 0,100 g |
| GLYCEROL | 8,000 g |
| EDETATE DISODIQUE PH EU | 0,200 g |
| MELANGE PIGMENTAIRE 17123 | 2,000 g |
| PARFUM | 0,100 g |
| AEROSIL R972 SILICE COLLOIDE | 0,100 à 1 g |
| BUTYL HYDROXY TOLUENE | 0,0100 g |
| EAU THERMALE D'AVENE qsp | 100,00 g |

On indique que :
- le MONOMULS 90L12 est du monolaurate de glycérol (HENKEL)
- le KELTROL T.F. est de la gomme xanthane (KELCO)
- le WITCONOL APM est du DPO 3 myrestil éther (WITCO)
- l'EUXYL K 400 est du dibromo dicyanobutane phénoxyéthanol (CALGON)
- l'EDETATE DISODIQUE PH EU est du EDTA 2 Na (BASF).

| **CREME SOLAIRE MINERALE** | |
|---|---|
| | Pourcentages |
| ABIL WE 09 | 4 |
| ELFACOS ST 37 | 0,6 |
| PARAFFINE LIQUIDE | 1 à 10,0 |
| TiO₂ PATE | 1 à 70 |
| Na Cl | 0,7 |
| CONSERVATEURS | QSP |
| OXYDE DE ZINC ENROBE | 1 à 25 |
| GUAR HYDROXYPROPYLTRIMONIUM | 0,7 |
| EAU | QSP 100 |
| BENTONE 38 - Quaternium 18 | 0,1 à 5 |
| AEROSIL R 972 | 0,1 à 5 |
| - ABIL WE 09 = Cétyl diméthicone Copolyol (GOLDSCHMIDT) (et) polyglycéryl 4 isostéarate (et) Hexyl laurate | |
| - ELFACOS ST 37 (AKZO) = PEG 22/Dodécyl glycol copolymers | |
| - Bentone 38 = Quaternium 18 - Hectorite (Rhéox) | |

| **CREME SOLAIRE E/H MINERALE ± ORGANIQUE** | |
|---|---|
| | Pourcentages |
| HOSTERACIN W/O | 10 à 15 |
| PATE DE TiO₂ | 1 à 70 |
| ANTARON WP 660 | 0,1 à 3 |
| ZnO ENROBE ET EMPATE A 50% | 1 à 50 |
| CONSERVATEURS | QSP |
| CINNAMATE | 0 à 10 |
| DIBENZOYLMETHANE | 0 à 4 |
| EAU | QSP 100 |
| GAMMA ORYZANOL | 0,2 |
| BENTONE 38 | 0,1 à 5 |
| AEROSIL R 972 | 0,1 à 6 |
| HOSTERACIN W/O (HOESCHT) : polyglycéryl 2 sesquiisostéarate (et) cire d'abeilles (et) Al minéral (et) stéarate de magnésium (et) stérate d'aluminium. | |
| ANTARON WP 660 (I.S.P.) : Polyvinylpyrolidone Tricantanyl | |

| **CREME SOLAIRE EXTREME MINERALE ET ORGANIQUE** | |
|---|---|
| | Pourcentages |
| DOW 3225 C | 5 à 15 |
| Pâte de TiO₂ dans FINSOLV TN et CETIOL VA50% | 0,1 à 60 |
| ZnO ENROBE EN POUDRE | 0,1 à 25 |
| CINNAMATE | 0 à 10 |
| DIBENZOMETHANE | 0 à 4 |
| MONOMULS 90 L 12 | 0,1 à 2 |
| MONOSTEARATE DE GLYCEROL | 0,1 à 2 |
| OZOKERITE | 0,1 à 3 |
| ACETATE DE TOCOPHEROL (Vitamine E) | 0,1 à 1 |
| WITCONOL APM | 0,1 à 1 |
| CONSERVATEUR | QSP |
| GLYCEROL | 1 à 10 |
| MELANGE D'OXYDE DE FER | 0,1 à 5 |
| SILICE COLLOIDALE | 0,1 à 5 |
| MONTMORILLONITE | 0,1 à 5 |
| EAU | QSP 100 |
| - MONOMULS 90 L 12 : monolaurate de glycérol (HENKEL) | |
| - WITCONOL APM : PPB3 - éther de myristel (WITCO) | |
| - DOW 3225C : cyclométhicone et diméthicone copolyol | |

| **CREME SOLAIRE E/SILICONE** | |
|---|---|
| | Pourcentages |
| ABIL EM 90 | 1 à 5 |
| ABIL WAX 9801 | 0,2 à 6 |
| DOW 345 | 0,1 à 10 |
| TiO₂ EMPATE A 30% | 0,1 à 70 |
| ZnO ENROBE POUDRE | 0,1 à 25 |
| EAU | QSP 100 |
| NaCl | 0,1 à 3 |
| ABIL EM 90 : cétyl diméthicone copolyol (GOLDSCHMIDT) | |
| ABIL WAX 9801 : cétyl diméthicone (GOLDSCHMIDT) | |
| DOW 345 : cyclométhicone (DOW CORNING) | |

| **CREME SOLAIRE E/H** | |
|---|---|
| | Pourcentages |
| ISOLAN GI 34 | 1 à 10 |
| TiO2 EMPATE A 50% | 1 à 60 |
| ZnO EN SUSPENSION A 60% | 1 à 50 |
| DOW 345 | 1 à 10 |
| VASELINE BLANCHE | 1 à 10 |
| EAU | QSP 100 |
| GLYCEROL | 1 à 10 |
| NaCl | 0,1 à 2 |
| ISOLAN GI 34 (GOLDSCHMIDT) : Polyglycéryl 4 - isostéarate. | |

| **LAIT E/HUILE SILICONE** | |
|---|---|
| | Pourcentages |
| Q2 5200 | 5 à 15 |
| DOW 345 | 1 à 10 |
| TiO₂EMPATE A 20% DANS LE CETIOL V | 1 à 80 |
| ZnO ENROBE EN POUDRE | 1 à 25 |
| CINNAMATE | 0 à 40 |
| DIBENZOYL METHANE | 0 à 4 |
| ANTORAN V 220 PVP copolymère | 0,1 à 3 |
| CIRE D'ABEILLE | 1 à 4 |
| EAU | QSP 100 |
| PARFUM | 0,1 à 1 |
| CONSERVATEURS | QSP |
| Q2 5200 (DOW CORNING) : laurylméthicone copolyol. | |

| **CREME SOLAIRE E/H SILICONE COMPACT** | |
|---|---|
| | Pourcentages |
| ABIL WE 09 | 0,5 à 5 |
| EAU | 1 |
| CIRE DE CARNAUBA | 1 à 10 |
| LANOLINE HYDROGENEE | 1 à 10 |
| ACETOXYSTEARATE | 1 à 10 |
| TiO₂ EMPATE à 50% dans le FINSOLV TN et le CETIOL V | 1 à 70 |
| ZnO ENROBE | 1 à 25 |
| BENZYLIDENE CAMPHRE | 0 à 8 |
| DIBENZOYL METHANE | 0 à 4 |
| BUTYL HYDROXY TOLUENE | 1 |
| OXYDE DE FER ENROBE | 0 à 2 |
| BENTONE 34 | 0,1 à 5 |
| AEROSIL | 0,1 à 5 |
| ABIL WE 09 : cétyl diméthicone copolyol (et) polyglycéril 4 isostéarate (et) hexyl laurate (GILDSCHMIDT). | |

| **LAIT SOLAIRE PROTECTION EXTREME MINERAL** | |
|---|---|
| | Pourcentages |
| DOW 3225 C | 5 à 15 |
| DECAMETHYL CYCLO PENTASILOXANE | 1 à 10 |
| PATE DE TITANE A 45% DANS LE MIGLYOL 812 | 1 à 70 |
| SPECTRAVEIL MOTG | 0,1 à 20 |
| BENTONE 38 | 0,1 à 5 |
| MONOSTEARATE DE GLYCEROL | 0,1 à 2 |
| ACETATE TOCOPHEROL | 0,1 à 1 |
| NaCl | 2 |
| CONSERVATEUR | QSP |
| GLYCEROL | 1 à 10 |
| EDTA | 0,1 à 0,2 |
| MELANGE D'OXYDE DE FER | 0,1 à 5 |
| PARFUM | |
| SILICE COLLOIDALE | 0,1 à 5 |
| BENTONE 38 | 0,1 à 5 |
| EAU MINERALE OU DEMINERALISEE | QSP 100 |

| **CREME SOLAIRE MINERALE + ORGANIQUE** | |
|---|---|
| | Pourcentages |
| ABIL WE 09 | 3 |
| TiO₂ PATE | 1 à 70 |
| ZnO ENROBE | 1 à 25 |
| CINNAMATE | 0,1 à 10 |
| DIBENZOYL METHANE | 0,1 à 4 |
| OZOKERITE | 1 |
| CONSERVATEURS | QSP |
| EAU | QSP 100 |
| BENTONE 34- Quaternarium 18 bentonite (...) | 0,1 à 5 |
| AEROBIL R 972 | 0,1 à 5 |

| **COMPACT ANHYDRE PROTECTEUR ET CORRECTEUR DE TEINT** | |
|---|---|
| | Pourcentages |
| TiO₂ EMPATE à 50% (dans le tridécyl stéarate et néopentyl glycol, caprate et dicaprylate et tridécyl trimellitate) | 1 à 70 |
| ZnO ENROBE ET EMPATE A 50% | 1 à 50 |
| CINNAMATE | 0 à 10 |
| DIBENZOYL METHANE | 0 à 4 |
| OXYDE DE FER | 12,7 |
| NEOPENTANOATE D'ISOSTEARYL | 5 |
| CARNAUBA | 2 |
| ULTRAMARINE | 1 |
| OZOKERITE | 1 |
| CONSERVATEURS | QSP |
| BENTONE 38 - Quaternarium 18 - Hectocrite | 0,1 à 5 |
| AEROSIL R 972 | 0,1 à 5 |

| **STICK ECRAN TOTAL** | |
|---|---|
| | Pourcentages |
| ACETATE DE TOCOPHEROL | 0,5 |
| CINNAMATE | 0 à 10 |
| DIBENZOYL METHANE | 0 à 14 |
| TiO₂ EMPATE à 40% dans FINSOLV TN | 1 à 70 |
| ZnO enrobé et empâté dans FINSOLV TN à 50% | 1 à 50 |
| ACETOXYSTEARATE D'ETHYLHEXYL | 1 à 6 |
| CARNAUBA | 1 à 15 |
| BUTYL HYDROXY TOLUENE | 0,01 |
| PARFUM | 0,1 à 2 |
| BENTONE 38 | 0,1 à 5 |
| AEROSIL R 972 | 0,1 à 5 |

## Revendications

1. Composition écran-solaire comprenant un mélange synergique de particules d'oxyde de titane et de particules d'oxyde de zinc, caractérisée par le fait que les particules d'oxyde de zinc et les particules d'oxyde de titane sont empâtées dans des huiles et/ou esters et/ou alcools gras et/ou éthers d'empâtage sélectionnés dans le but d'obtenir :
- un point de mouillage des particules d'oxyde de titane empâtées et un point de mouillage des particules d'oxyde de zinc empâtées, environ inférieurs à 5,
- un point de fluidité des particules d'oxyde de titane empâtées et d'un point de fluidité des particules d'oxyde de zinc empâtées, environ inférieurs à 30.
le point de mouillage et le point de fluidité des particules empâtées désignant respectivement la quantité en poids de vaseline à ajouter à un mélange de 9 g de particules et 7 g d'huile d'empâtage, pour obtenir le mouillage complet des poudres, respectivement une pâte fluide.

2. Composition écran-solaire selon la revendication 1, caractérisée par le fait que les particules d'oxyde de titane présentent une taille inférieure ou égale à 20 ± 5 nm et les particules d'oxyde de zinc, une taille supérieure ou égale à 60 ± 5 nm.

3. Composition écran-solaire selon l'une quelconque des revendications précédentes, caractérisée par le fait que les particules d'oxyde de titane présentent une taille moyenne égale à 20 nm et les particules d'oxygène, une taille moyenne égale à 60 nM.

4. Composition écran-solaire selon l'une quelconque des revendications précédentes, caractérisée par le fait que les huiles et/ou esters et/ou alcools gras et/ou éthers d'empâtage sont sélectionnés dans le groupe comprenant le décyl oléate, un C₁₂₋₁₅ alkyl benzoate, l'octyl dodécanol, l'octyl dodécyl néopentanoate, le gylcéryl oléate, le propylène glycol, le propylène glycol dioctanoate, les triglycérides caprique/caprylique, le cétéaryl octanoate, l'octyl palmitate, l'isoarachidyl néopentanoate, le dioctyl maléate, le dicapryléther, ainsi que leurs mélanges.

5. Composition écran-solaire selon l'une quelconque des revendications précédentes, caractérisée par le fait que l'huile et/ou l'ester et/ou l'alcool gras et/ou l'éther d'empâtage des particules d'oxyde de titane est constitué par un mélange de décyl oléate et d'un C₁₂₋₁₅ alkyl benzoate et l'huile et/ou l'ester et/ou l'alcool gras et/ou l'éther d'empâtage des particules d'oxyde de zinc comprend des triglycérides caprique/caprylique par exemple.

6. Composition écran-solaire selon l'une quelconque des revendications précédentes, caractérisée par le fait que l'oxyde de titane est présent à une concentration relative en poids comprise entre 20 et 60% dans l'empâtage et au maximum 25 % dans le produit fini.

7. Composition écran-solaire selon l'une quelconque des revendications précédentes, caractérisée par le fait que l'oxyde de zinc est présent à une concentration relative en poids comprise entre 20 et 60 % dans l'empâtage et au maximum 30 % dans le produit fini.

8. Composition écran-solaire selon l'une quelconque des revendications précédentes, caractérisée par le fait qu'elle comporte en outre des particules d'oxyde de fer de taille moyenne égale à 1 à 10 µm, leur proportion relative en poids étant comprise entre 0,1 et 3 %.

9. Procédé de fabrication d'une composition écran-solaire selon l'une quelconque des revendications précédentes, caractérisé par le fait qu'il comporte une étape préliminaire consistant à :
- empâter des particules d'oxyde de titane dans au moins une huile et/ou un ester et/ou un alcool gras et/ou un éther d'empàtage, afin d'obtenir des particules d'oxyde de titane empâtées, et
- empâter des particules d'oxyde de zinc dans une au moins une huile et/ou un ester et/ou un alcool gras et/ou un éther d'empâtage, afin d'obtenir des particules d'oxyde de zinc empâtées, éventuellement
- mélanger les particules d'oxyde de titane empâtées et les particules d'oxyde de zinc empâtées, afin d'obtenir une pâte destinée à être incorporée dans ladite composition.

10. Procédé selon la revendication 9, caractérisé par le fait que l'huile et/ou l'ester et/ou l'alcool gras et/ou l'éther d'empâtage des particules d'oxyde de titane et l'huile et/ou l'ester et/ou l'alcool gras et/ou l'éther d'empâtage des particules d'oxyde de zinc sont sélectionnés indépendamment l'un de l'autre, afin d'obtenir :
- un point de mouillage des particules d'oxyde de titane empâtées et un point de mouillage des particules d'oxyde de zinc empâtées environ inférieurs à 5, et
- un point de fluidité des particules d'oxyde de titane empâtées et un point de fluidité des particules d'oxyde de zinc empâtées environ inférieurs à 30.

11. Procédé selon l'une quelconque des revendications 9 ou 10, caractérisé par le fait que la composition se présente sous la forme d'une émulsion eau dans huile, en particulier une émulsion eau dans huile à base de silicone.

12. Utilisation cosmétique d'une composition écran-solaire selon l'une quelconque des revendications 1 à S, pour la protection de la peau contre tous les effets des radiations solaires UV B et UV A à court ou à long terme.

13. Utilisation d'une composition écran-solaire selon l'une quelconque des revendications 1 à 8, pour la fabrication de médicaments destinés à être utilisés pour des traitements dermatologiques d'affections telles que la Lucite Estivale Bénigne (L.E.B.).

## Patentansprüche

1. Sonnenschutzmittel aus einer synergistischen Mischung von Titanoxidteilchen und Zinkoxidteilchen dadurch gekennzeichnet, daß die Zinkoxidteilchen und die Titanoxidteilchen in pastierenden Ölen und/oder Estern und/oder Fettalkoholen und/oder Äthern verpastet sind, die so gewählt sind, daß
- der Benetzungspunkt der verpasteten Titanoxidteilchen und der Benetzungspunkt der verpasteten Zinkoxidteilchen unter etwa 5 liegt,
- der Fluiditätspunkt der verpasteten Titanoxidteilchen und der Fluiditätspunkt der verpasteten Zinkoxidteilchen unter etwa 30 liegt,
wobei der Benetzungspunkt und der Fluiditätspunkt der verpasteten Teilchen jeweils die Gewichtsmenge an Vaseline bezeichnet, die eine Mischung von 9 g Teilchen und 7 g Verpastungsöl zuzusetzen ist, um eine vollständige Benetzung der Pulver bzw. eine flüssige Paste zu erhalten.

2. Sonnenschutzmittel nach Anspruch 1, dadurch gekennzeichnet, daß die Titanoxidteilchen eine Größe von weniger oder gleich 20 ± 5 nm haben und daß die Zinkoxidteilchen eine Größe von mehr als oder gleich 60 ± 5nm haben.

3. Sonnenschutzmittel nach einem der vorhergehenden Ansprüche, dadurch gekennzeichnet, daß die Titanoxidteilchen eine mittlere Größe von 20 nm haben und daß die Zinkoxidteilchen eine mittlere Größe von 60 nm haben.

4. Sonnenschutzmittel nach einem der vorhergehenden Ansprüche, dadurch gekennzeichnet, daß die pastierenden öle und/oder Ester und/oder Fettalkohole und/oder Äther aus einer Gruppe gewählt sind, die Decyloleat, ein C₁₂₋₁₅ Alkylbenzoat, Octyldodecanol, Octyldodecylneopentanoat, Glyceryloleat, Propylenglykol, Propylenglykoldioctanoate, caprikische/caprylische Triglyceride, Cetearyloctanoat, Octylpalmitat, Isoarachidylneopentanoat, Dioctylmaleat, Dicaprylether sowie ihre Mischungen umfaßt.

5. Sonnenschutzmittel nach einem der vorhergehenden Ansprüche, dadurch gekennzeichnet, daß das Öl und/oder der Ester und/oder der Fettalkohol und/oder der Äther zum Verpasten der Titanoxidteilchen aus einer Mischung von Decyloleat und einem C₁₂₋₁₅ Alkylbenzoat besteht und daß das Öl und/oder der Ester und/oder der Fettalkohol und/oder der Äther zum Verpasten der Zinkoxidteilchen beispielsweise caprikische/caprylische Triglyceride umfaßt.

6. Sonnenschutzmittel nach einem der vorhergehenden Ansprüche, dadurch gekennzeichnet, daß das Titanoxid in einer relativen Gewichtskonzentration zwischen 20 und 60 % in der Paste und maximal 25 % im endgültigem Produkt vorliegt.

7. Sonnenschutzmittel nach einem der vorhergehenden Ansprüche, dadurch gekennzeichnet, daß das Zinkoxid in einer relativen Gewichtskonzentration zwischen 20 und 60 % in der Paste und maximal 30 % im endgültigem Produkt vorliegt.

8. Sonnenschutzmittel nach einem der vorhergehenden Ansprüche, dadurch gekennzeichnet, daß es weiterhin Eisenoxidteilchen mit einer mittleren Größe von 1 bis 10 *µ*m enthält, deren relativer Gewichtsanteil zwischen 0,1 und 3 % liegt.

9. Verfahren zum Herstellen eines Sonnenschutzmittels nach einem der vorhergehenden Ansprüche, dadurch gekennzeichnet, daß es einen vorausgehenden Schritt umfaßt, der darin besteht, daß
- Titanoxidteilchen wenigstens in einem pastierenden Öl und/oder einem pastierenden Ester und/oder einem pastierenden Fettalkohol und/oder einem pastierenden Äther verpastet werden, um verpastete Titanoxidteilchen zu erhalten, und
- Zinkoxidteilchen in wenigstens in einem pastierenden Öl und/oder einem pastierenden Ester und/oder einem pastierenden Fettalkohol und/oder einem pastierenden Äther verpastet werden, um verpastete Zinkoxidteilchen zu erhalten, wobei ggf.
- die verpasteten Titanoxidteilchen und die verpasteten Zinkoxidteilchen gemischt werden, um eine Paste zu erhalten, die dazu bestimmt ist, dem besagten Mittel zugegebenen zu werden.

10. Verfahren nach Anspruch 9, dadurch gekennzeichnet, daß das pastierende Öl und/oder der pastierende Ester und/oder der pastierende Fettalkohol und/oder der pastierende Äther für die Titanoxidteilchen und das pastierende Öl und/oder der pastierende Ester und/oder der pastierende Fettalkohol und/oder der pastierende Äther für die Zinkoxidteilchen unabhängig voneinander so gewählt werden, daß
- der Benetzungspunkt der verpasteten Titanoxidteilchen und der Benetzungspunkt der verpasteten zinkoxidteilchen unter etwa 5 liegt und
- der Fluiditätspunkt der verpasteten Titanoxidteilchen und der Fluiditätspunkt der verpasteten Zinkoxidteilchen unter etwa 30 liegt.

11. Verfahren nach Anspruch 9 oder 10, dadurch gekennzeichnet, daß das Mittel in Form einer Wasser-in-Ö1-Emulsion insbesondere einer Wasser-in-Öl-Emulsion auf Silikonbasis vorliegt.

12. Kosmetische Verwendung eines Sonnenschutzmittels nach einem der Ansprüche 1 bis 8 zum Schützen der Haut gegenüber Einflüssen der UVB und UVA Sonnenstrahlung und zwar kurzfristig oder langfristig.

13. Verwendung eines Sonnenschutzmittels nach einem der Ansprüche 1 bis 8 zur Herstellung von Medikamenten, die dazu bestimmt sind, bei dermatologischen Behandlungen von Erkrankungen wie beispielsweise Lucite Estival Benigne (L.E.B.) verwandt zu werden.

## Claims

1. Sunscreen composition comprising a synergic mixture of titanium oxide particles and of zinc oxide particles, characterized in that the zinc oxide particles and the titanium oxide particles are incorporated in paste-forming oils and/or esters and/or fatty alcohols and/or ethers selected with the aim of producing:
- a wetting point for the incorporated titanium oxide particles and a wetting point for the incorporated zinc oxide particles which are less than approximately 5,
- a flow point for the incorporated titanium oxide particles and a flow point for the incorporated zinc oxide particles which are less than approximately 30,
the wetting point and the flow point for the incorporated particles respectively denoting the amount by weight of petrolatum to be added to a mixture of 9 g of particles and 7 g of paste-forming oil in order to obtain complete wetting of the powders and a fluid paste, respectively.

2. Sunscreen composition according to Claim 1, characterized in that the titanium oxide particles exhibit a size of less than or equal to 20 ± 5 nm and the zinc oxide particles a size of greater than or equal to 60 ± 5 nm.

3. Sunscreen composition according to any one of the preceding claims, characterized in that the titanium oxide particles exhibit a mean size of 20 nm and the zinc oxide particles a mean size of 60 nm.

4. Sunscreen composition according to any one of the preceding claims, characterized in that the paste-forming oils and/or esters and/or fatty alcohols and/or ethers are selected from the group comprising decyl oleate, a C₁₂₋₁₅ alkyl benzoate, octyldodecanol, octyldodecyl neopentanoate, glyceryl oleate, propylene glycol, propylene glycol dioctanoate, capric/caprylic triglycerides, cetearyl octanoate, octyl palmitate, isoarachidyl neopentanoate, dioctyl maleate, dicapryl ether and their mixtures.

5. Sunscreen composition according to any one of the preceding claims, characterized in that the oil and/or the ester and/or the fatty alcohol and/or the ether for incorporating the titanium oxide particles is composed of a mixture of decyl oleate and of a C₁₂₋₁₅ alkyl benzoate and the oil and/or the ester and/or the fatty alcohol and/or the ether for incorporating the zinc oxide particles comprises capric/caprylic triglycerides, for example.

6. Sunscreen composition according to any one of the preceding claims, characterized in that the titanium oxide is present at a relative concentration by weight of between 20 and 60% in the incorporation product and of at most 25% in the finished product.

7. Sunscreen composition according to any one of the preceding claims, characterized in that the zinc oxide is present at a relative concentration by weight of between 20 and 60% in the incorporation product and of at most 30% in the finished product.

8. Sunscreen composition according to any one of the preceding claims, characterized in that it additionally contains iron oxide particles with a mean size of 1 to 10 µm, their relative proportion by weight being between 0.1 and 3%.

9. Method for manufacturing a sunscreen composition according to any one of the preceding claims, characterized in that it contains a preliminary stage consisting in:
- incorporating titanium oxide particles in at least one paste-forming oil and/or ester and/or fatty alcohol and/or ether, in order to obtain incorporated titanium oxide particles, and
- incorporating zinc oxide particles in at least one paste-forming oil and/or ester and/or fatty alcohol and/or ether, in order to obtain incorporated zinc oxide particles, optionally
- mixing the incorporated titanium oxide particles and the incorporated zinc oxide particles, in order to obtain a paste intended to be incorporated in the said composition.

10. Method according to Claim 9, characterized in that the oil and/or the ester and/or the fatty alcohol and/or the ether for incorporating the titanium oxide particles and the oil and/or the ester and/or the fatty alcohol and/or the ether for incorporating the zinc oxide particles are selected independently of one another, in order to obtain:
- a wetting point for the incorporated titanium oxide particles and a wetting point for the incorporated zinc oxide particles which are less than approximately 5, and
- a flow point for the incorporated titanium oxide particles and a flow point for the incorporated zinc oxide particles which are less than approximately 30.

11. Method according to either one of Claims 9 and 10, characterized in that the composition is provided in the form of a water-in-oil emulsion, in particular a water-in-oil emulsion in which the oil is silicone-based.

12. Cosmetic use of a sunscreen composition according to any one of Claims 1 to 8, for protecting the skin against all the short- or long-term effects of UV B and UV A solar radiation.

13. Use of a sunscreen composition according to any one of Claims 1 to 8, for the manufacture of medicaments intended to be used in dermatological treatments of complaints such as Benign Estival Photodermatosis (B.E.P.).
